# EUROPÄISCHE PATENTSCHRIFT

(11) **EP 1 453 775 B1**
(45) Veröffentlichungstag und Bekanntmachung des Hinweises auf die Patenterteilung: **26.07.2006**
(21) Anmeldenummer: 02801869.5
(22) Anmeldetag: 20.09.2002
(51) Int. Cl.: C07C 5/22, C07C 11/08, C07C 11/10, C07C 11/107

(54) **HYDROISOMERISIERUNG VON OLEFINEN MIT 4-6 KOHLENSTOFFATOMEN**
HYDROISOMERISATION OF OLEFINS COMPRISING BETWEEN 4 AND 6 CARBON ATOMS
HYDRO-ISOMERISATION D'OLEFINES COMPORTANT 4-6 ATOMES DE CARBONE

(30) Priorität: 25.10.2001 DE 10152842
(43) Veröffentlichungstag der Anmeldung: 08.09.2004
(73) Patentinhaber: Oxeno Olefinchemie GmbH, 45772 Marl (DE)
(72) Erfinder: BECKMANN, Andreas, 45657 Recklinghausen (DE); RIX, Armin, 45770 Marl (DE); KNIPPENBERG, Udo, 45772 Marl (DE); NIERLICH, Franz, 45768 Marl (DE); BÜSCHKEN, Wilfried, 45721 Haltern (DE); DÜSSEL, Ralf, Waterford, NY 12188 (US)
(74) Vertreter: Lang, Arne
(86) Internationale Anmeldenummer: PCT/EP2002/010571
(87) Internationale Veröffentlichungsnummer: WO 2003/035587

(56) Entgegenhaltungen:
- DE-A- 2 728 218
- DE-A- 19 957 173
- US-A- 3 531 545
- US-A- 3 749 752

## Beschreibung

Die Erfindung betrifft ein Verfahren zur Isomerisierung von Monoolefinen mit 4 bis 6 Kohlenstoffatomen und endständigen Doppelbindungen (α-Olefine) zu den entsprechenden Monoolefinen mit innenständigen Doppelbindungen. Gegebenenfalls enthaltene mehrfach ungesättigte Kohlenwasserstoffe werden mit hoher Selektivität zu Monoolefinen hydriert und zu den entsprechenden Monoolefinen mit innenständiger Doppelbindung isomerisiert. Die Isomerisierung beinhaltet eine Verschiebung der olefinischen Doppelbindung und von Wasserstoffatomen unter Beibehaltung des Kohlenstoffgerüstes und wird im Folgenden kurz Hydroisomerisierung genannt.
Die Erfindung betrifft weiterhin die Verwendung der durch die erfindungsgemäße Hydroisomerisierung hergestellten Monoolefine, insbesondere zur Herstellung höherer Oligomerer und zur Herstellung von Alkylatbenzin.

Die Hydroisomerisierung ist ein wichtiger Verfahrensschritt bei der Aufarbeitung von C₄ - C₆₋olefinhaltigen Gemischen, wenn Olefine mit vorwiegend innenständigen Doppelbindungen die Hauptprodukte sein sollen. So werden zum Beispiel C₄-olefinhaltige Gemische je nach den gewünschten Zielprodukten unterschiedlich aufgearbeitet. Der erste Schritt, den dabei alle Aufarbeitungsvarianten gemeinsam haben, ist die Entfernung des größten Teils des Butadiens. Kann Butadien gut vermarktet werden oder besteht ein Eigenverbrauch, wird es durch Extraktion oder Extraktivdestillation abgetrennt. Im anderen Fall wird es bis zu einer Restkonzentration von circa 2000 ppm selektiv zu linearen Butenen hydriert. Zurück bleibt in beiden Fällen ein Kohlenwasserstoffgemisch (sogenanntes Raffinat I oder hydriertes Crack-C₄), das neben den gesättigten Kohlenwasserstoffen n-Butan und Isobutan die Olefine Isobuten, 1-Buten und 2-Butene (cis und trans) enthält. Wenn 1-Buten eines der Zielprodukte ist, wird wie folgt weiter vorgegangen: aus Raffinat I oder hydriertem Crack-C₄ wird durch chemische Umsetzung Isobuten entfernt. Dabei ist das technisch bedeutendste Verfahren die Umsetzung des Isobutens mit Methanol zu Methyl-tert.-butylether (MTBE), das unter anderem als Kraftstoffzusatz große Verwendung findet. Andere Möglichkeiten sind die Umsetzung des Isobutens zum tertiären Butanol (TBA) oder die sauer katalysierte Oligomerisierung des Isobutens zu Diisobuten und höheren Oligomeren. Aus dem verbleibenden isobutenfreien C₄₋Schnitt (sogenanntes Raffinat II), der die linearen Olefine und gesättigten Kohlenwasserstoffe enthält, werden die noch vorhandenen Butadienmengen durch selektive Hydrierung (SHP) entfernt (Restkonzentration unter 5 ppm). Aus diesem Gemisch wird 1-Buten und Isobutan vollständig oder teilweise destillativ abgetrennt. Das restliche Gemisch aus linearen Butenen und gesättigten Kohlenwasserstoffen kann, beispielsweise nach dem sogenannten Octol-Verfahren, oligomerisiert werden, wobei Di-n-Buten als Hauptprodukt entsteht.

Ist dagegen die Herstellung von Isobuten und 2-Buten beziehungsweise ein Gemisch linearer Butene mit hohem 2-Butengehalt das Ziel, wird folgender Aufarbeitungsgang bevorzugt: C₄₋Ströme, die typischerweise nicht mehr als 1 % Butadien enthalten (C₄-Strom aus FCC (Fluid catalytic cracker), Raffinat I oder hydriertes Crack-C₄), werden hydriert und hydroisomerisiert, das heißt, es wird (noch) vorhandenes Butadien bis zu einem Restgehalt von unter 5 ppm selektiv hydriert und gleichzeitig 1-Buten zu 2-Butenen isomerisiert. Die Gleichgewichtslage zwischen 1-Buten und den beiden 2-Butenen zusammen liegt zum Beispiel bei 80 °C bei circa 1 : 17, also weit auf der Seite der 2-Butene. Aus dem Hydroisomerisierungsgemisch kann wegen der geringen Siedepunktsdifferenzen nur ein Gemisch aus Isobuten, 1-Buten und Isobutan als Kopfprodukt gewonnen werden, das in üblicher Weise aufgearbeitet werden kann. Als Sumpfprodukt wird ein Isobuten-freies Gemisch erhalten. Dieses Gemisch ist ein hervorragend geeignetes Edukt für die Herstellung von Oligomeren, vorzugsweise von C₈₋Olefinen und insbesondere Di-n-Buten.
Für die Oligomerisierung gibt es im Prinzip drei Verfahrensvarianten. Lange bekannt ist die Oligomerisierung an sauren Katalysatoren, wobei technisch zum Beispiel Zeolithe oder Phosphorsäure auf Träger eingesetzt werden. Hierbei werden Isomerengemische von verzweigten Olefinen erhalten, die im Wesentlichen Dimethylhexene darstellen (WO 92/13818). Ein ebenfalls weltweit ausgeübtes Verfahren ist die Oligomerisierung mit löslichen Nickel-Komplexen, bekannt als DIMERSOL-Verfahren (B. Cornils, W. A. Herrmann, Applied Homogenous Catalysis with Organometallic Compounds, Seite 261 - 263, Verlag Chemie 1996). Schließlich ist noch die Oligomerisierung an Nickel-Festbett-Katalysatoren zu erwähnen, wie zum Beispiel das Verfahren der OXENO GmbH. Das Verfahren hat Eingang in die Literatur als OCTOL-Prozess (Hydrocarbon Process., Int. Ed. (1986) 65 (2. Sect.1), Seite 31 - 33) gefunden. DE2728218 offenbart ein Verfahren ein Isomerisierung von 1-Buten in 2-Buten.

Die so erhaltenen Dibutene sind begehrte Einsatzstoffe in der chemischen Industrie. So können beispielsweise durch Hydroformylierung um ein Kohlenstoffatom längere Aldehyde gewonnen werden - im Fall von Dibuten somit C₉-Aldehyde - die ihrerseits wieder für wichtige technische Produkte Einsatz finden. Beispiele sind die Hydrierung der Aldehyde zu den Alkoholen und ihre Umsetzung mit Carbonsäuren beziehungsweise Carbonsäureanhydriden zu Estern. So führt die Veresterung der Alkohole mit Phthalsäureanhydrid zu Diisononylphthalaten, die sehr begehrte Weichmacher in der kunststoffverarbeitenden Industrie sind. Technisch wichtig und ausgeübt ist des Weiteren die Oxidation der Aldehyde zu den entsprechenden Carbonsäuren, die unter anderem zu öllöslichen Metallsalzen umgesetzt werden. Diese werden beispielsweise als Trocknungsbeschleuniger für Lacke (Sikkative) oder Stabilisatoren für Polyvinylchlorid eingesetzt.

Eine weitere beispielhafte technische Anwendung ist die Umsetzung von Olefinen (Dibutenen) unter Katalyse von starken Säuren mit Kohlenmonoxid und Wasser zu den um ein Kohlenstoffatom längeren Carbonsäuren, die unter dem Namen KOCH-Reaktion Eingang in die Literatur gefunden hat. Hier werden tertiäre verzweigte Carbonsäuregemische erhalten, die wegen ihrer verzweigten Natur wiederum sehr gut zur Herstellung der zuvor erwähnten Metallsalze geeignet sind. Ein besonders wichtiger Einsatz der tertiären Carbonsäuren besteht in der Umsetzung mit Acetylen zu Vinylestern, die als Comonomere zur inneren Weichmachung von Polymeren dienen. Copolymere von Vinylestem tertiärer Carbonsäuren mit Vinylacetat sind beispielsweise die Basis für wasserdispergierbare umweltfreundliche Farben und Lacke und energiesparende Wärmeschutzverputze von Gebäuden.

Die bei der Oligomerisierung entstehenden höheren Oligomeren werden ebenfalls für chemische Synthesen eingesetzt. So wird aus Tributen durch Hydroformylierung und nachfolgende Hydrierung ein Gemisch isomerer C₁₃-Alkohole gewonnen, das als Vorstufe für die Herstellung von Detergentien und Weichmachern Verwendung findet. Die Hydrocarboxylierung ergibt tertiäre C₁₃-Carbonsäuren, die für ähnliche Anwendungen wie die entsprechenden C₉-Carbonsäuren eingesetzt werden können.
Das Restgas der Oligomerisierung besteht aus n-Butan und geringen Mengen an Butenen, die durch Hydrierung entfernt werden. Das hochreine n-Butan wird beispielsweise als Treibmittel für die Herstellung von Aerosolen eingesetzt.
Bei der Hydrierung und Hydroisomerisierung von Olefingemischen besteht die Schwierigkeit darin, die Hydrierung derart zu steuern, dass die mehrfach ungesättigten Olefine, die nur in geringen Konzentrationen vorliegen, zu den entsprechenden Monoolefinen umgesetzt, jedoch nicht die in hohen Konzentrationen vorliegenden Monoolefine zu den gesättigten Kohlenwasserstoffen hydriert werden.

Für die Hydrierung und Hydroisomerisierung von C₄-Olefingemischen bedeutet dies speziell, Butadien ohne Verluste an Butenen unter gleichzeitiger Isomerisierung der linearen Butene zu hydrieren.

Die Patentschrift US 4 849 576 beschreibt ein Verfahren zur Isomerisierung von Olefinen, insbesondere von n-Butenen, in einem Einsatzgemisch, das geringe Mengen an Schwefelverbindungen enthält. Die Isomerisierung erfolgt am Kontakt Pd/Al₂O₃ in Gegenwart von Wasserstoff. Um seine Standzeit zu erhöhen, wird ein Schutzkontakt (SnO₂ auf Al₂O₃) vorgeschaltet, der zumindestens einen Teil der Schwefelverbindungen zurückhält. Über die Hydrierung von Dienen, die möglicherweise im Einsatzprodukt vorhanden sind, werden in dieser Schrift keine Angaben gemacht. Es geht aus der Schrift auch nicht hervor, ob Olefine zu gesättigten Kohlenwasserstoffen hydriert werden.

In der Patentschrift US 3 531 545 wird die Isomerisierung von Olefinen an einem Palladium-Kontakt in Gegenwart von Wasserstoff und Schwefelverbindungen beschrieben. Dabei sollen Diene, Polyolefine oder Acetylenderivate vor der Isomerisierung entfernt werden, da sie den Prozess stören (Spalte 1, Zeilen 49 - 57).

Beide genannten Verfahren sind also ausschließlich Isomerisierungsverfahren ohne Selektivhydrierung der mehrfach ungesättigten Kohlenwasserstoffe.

Verfahren zur Entfernung von Schwefelverbindungen und Dienen unter gleichzeitiger Isomerisierung der vorhandenen Olefine aus C₃ - C₁₂-Erdölfraktionen und Gewinnung eines C₃ - C₅- oder C₄ - C₅-Schnitts werden von Hearn et al. in US 5 510 568 , US 5 595 634 und WO 98/12158 dargelegt. Nach US 5 510 568 erfolgt die Aufarbeitung in einer Reaktivkolonne, die einen Palladium-Kontakt enthält, in Gegenwart von Wasserstoff. Dabei werden C₃ - C₅₋Schnitte gewonnen, die noch Diene enthalten. Der Diengehalt beträgt in Beispiel 1 10 ppm und in Beispiel 2 50 ppm. Laut US 5 595 634 werden H₂S und C₃-Kohlenwasserstoffe in einer Vorkolonne als Kopfprodukte abgetrennt. Das Sumpfprodukt wird mit Wasserstoff in eine Reaktivkolonne geleitet. In dieser Kolonne sind ein Nickel-Kontakt und ein Palladium-Kontakt in getrennten Betten installiert. Der Nickel-Kontakt befindet sich im weiter unten in der Kolonne installierten Bett. Die Addition der im Edukt vorhandenen Mercaptane an olefinische Doppelbindungen geschieht hauptsächlich am Nickel-Kontakt, die Isomerisierung und Selektivhydrierung vorwiegend am Palladium-Kontakt. Als Kopfprodukt fällt ein C₄ - C₅₋Schnitt an. Darin ist nach beiden Beispielen der Diengehalt 46 ppm. Nach WO 98/12158 wird das Edukt zunächst durch einen Vorreaktor geleitet, der einen Ni/Al₂O₃-Kontakt enthält. Darin addiert sich ein Teil der Mercaptane an Diene. Der Reaktoraustrag wird zusammen mit Wasserstoff in eine Reaktivkolonne mit einem Pd/Al₂O₃-Katalysator geleitet. Eine C₃ - C₅₋Fraktion fällt als Kopfprodukt an. Dieser Schnitt ist weder Dien- noch Mercaptan-frei. Gehalte dieser Verbindungen werden nicht offenlegt. Es wird nur unbestimmt von niedrigerem Diengehalt und reduziertem Mercaptangehalt im Vergleich zum Edukt berichtet.

In der Patentschrift US 5 759 386 wird eine Leichtbenzinfraktion, die geringe Mengen an Dienen (ca. 0,6 % nach Beispiel 1) und Schwefelverbindungen (10 - 350 ppm S) enthalten, aufgearbeitet. Im ersten Schritt wird das Edukt an einem Nickel-Kontakt in Gegenwart von Wasserstoff umgesetzt. Dabei reagieren Mercaptane zu Thioether, überschüssige Diene werden selektiv hydriert und die Olefine isomerisiert. Der Reaktoraustrag wird in eine Kopffraktion, die C₃ - C₅-Kohlenwasserstoffe enthält, und eine Sumpffraktion mit den höher siedenden Stoffen aufgetrennt. Auch bei diesen Verfahren ist die Leichtsiederfraktion nicht frei von Dienen und Schwefelverbindungen. Wie aus dem Beispiel 1 hervorgeht, beträgt der Thioethergehalt 118 ppm und der Diengehalt 75 ppm.
In der europäischen Patentanmeldung EP-A-0 556 025 wird ein Verfahren zur Entfernung von Dienen aus einer Leichtbenzinfraktion (beispielsweise mit 5 % C₄- , 69 % C₅- und 25,6 % C₆₋Kohlenwasserstoffen) und gleichzeitiger Isomerisierung der Monoolefine offenbart. Dabei wird das Leichtbenzin zusammen mit Wasserstoff in eine Destillationskolonne, die einen Pd/Al₂O₃₋Kontakt enthält, geleitet. Wasserstoff und Leichtsieder (0,5 - 1 % des Edukts) fallen als Kopfprodukte an. Das Sumpfprodukt hat dabei einen Restdiengehalt von 40 ppm. Der Verlust an Monoenen beträgt 1,9 %.
In GB 1 110 826 wird die Isomerisierung von 1-Buten zu den 2-Butenen unter gleichzeitiger Entfernung von Butadien (unter 5 % im Edukt) durch Selektivhydrierung zu linearen Butenen beschrieben. Als Katalysator wird ein geschwefelter Nickel-Trägerkontakt (Ni auf Sepiolith) eingesetzt. Dabei stellt sich bei 100 °C ein 2-Buten : 1-Buten-Verhältnis von 10,6 : 1 ein, also ein Verhältnis, das weit vom thermodynamischen Gleichgewicht von circa 16 : 1 entfernt ist.

In der Patentschrift US 4 132 745 wird ein Verfahren zur Isomerisierung von 1-Buten zu 2-Butenen unter gleichzeitiger Hydrierung geringer im Edukt enhaltener Butadienmengen offengelegt. Als Katalysator wird ein geschwefelter Palladium-Kontakt verwendet. Nachteile dieses Verfahren bestehen darin, dass ein Teil der Olefine durch Überhydrierung zu Butan verlorengeht und dass nicht mehr als 10 ppm Schwefel im Edukt toleriert werden können. Weiterhin enthält das Produktgemisch Schwefelverbindungen.

In der europäischen Patentanmeldung EP-A-0 636 677 wird zur Isomerisierung von linearen Butenen und zur Entfernung von geringen Butadienmengen ebenfalls ein geschwefelter Pd/Al₂O₃-Kontakt verwendet. Dabei werden zwar die Diene hydriert, jedoch das thermodynamische Gleichgewicht der linearen Butene bei weitem nicht eingestellt.

In der europäischen Patentanmeldung EP-A-0 288 362 werden Verfahren zur Hydrierung und Hydroisomerisierung von C₄-Schnitten, die 20 ppm Schwefel enthalten, beschrieben. Die Umsetzung erfolgt an zwei hintereinander geschalteten Kontakten (Katalysatoren). Der erste Kontakt enthält als aktive Komponenten Palladium und mindestens ein weiteres Metall, das Gold und/oder Platin sein kann. Der zweite Kontakt (Katalysator) enthält Palladium. Die Umsetzung kann mit reinem Wasserstoff oder mit Wasserstoff mit einem Gehalt bis zu 10 000 ppm Schwefelwasserstoff erfolgen. Nachteilig bei diesen Verfahren ist, dass nach den Beispielen mit praktisch vollständigem Butadienumsatz 1-Buten nur zu 80 % der theoretisch möglichen Menge zu 2-Buten isomerisiert wird. Darüber hinaus sind die entstehenden Produkte schwefelhaltig. Eine Methode zur Entfernung der Schwefelverbindungen wird nicht genannt.

In den bekannten Verfahren moderieren Schwefelverbindungen im Edukt die Aktivität der verwendeten Katalysatoren. Deshalb eignen sie sich besonders gut für die Hydroisomerisierung von schwefelreichen Kohlenwasserstoffströmen. Diese Verfahren haben jedoch den Nachteil, das sie schwefelhaltige Produkte liefern. Darüber hinaus sind häufig die Restdiengehalte und die Verluste an Monoolefinen durch Überhydrierung relativ hoch. Außerdem wird das thermodynamische Gleichgewicht zwischen Olefinen mit endständigen und innenständigen Doppelbindungen nicht immer eingestellt.

Es bestand daher die Aufgabe, ein Hydroisomerisierungsverfahren für C₄- C₆-Olefine zu entwickeln, bei dem gegebenenfalls in einer Konzentration von bis zu 5 % vorhandene mehrfach ungesättigte Kohlenwasserstoffe gleichzeitig selektiv zu Monoolefinen hydriert werden, wobei das Verfahren die oben geschilderten Nachteile nicht aufweist und für die Umsetzung von vorzugsweise schwefelarmen Edukten zu praktisch schwefelfreien Produkten geeignet ist, die insbesondere zur Oligomerisierung eingesetzt werden können oder zur Herstellung von Alkylatbenzin geeignet sind.

Erfindungsgemäß wird diese Aufgabe dadurch gelöst, dass C₄- bis C₆-Olefine oder ein olefinhaltiger Schnitt mit C₄- bis C₆-Olefinen an einem Kontakt, der ein Element der achten Nebengruppe des Periodensystems der Elemente enthält, in Gegenwart mindestens einer zugesetzten Schwefelverbindung, die mindestens 4 Kohlenstoffatome enthält und leicht abtrennbar ist, hydroisomerisiert wird oder dass ein Gemisch von C₄- bis C₆-Olefinen mit darin bis zu 5 %, vorzugsweise bis zu 2 %, enthaltenen mehrfach ungesättigten Kohlenwasserstoffen in Gegenwart mindestens einer zugesetzten Schwefelverbindung, die die oben genannten Bedingungen erfüllt, hydroisomerisiert und hydriert wird und die Schwefelverbindungen aus dem Produkt abgetrennt und gegebenenfalls wieder ins Edukt zurückgeführt werden.
Die Erfindung betrifft demnach ein Verfahren zur Herstellung von C₄- bis C₆-Olefinen oder eines Gemisches von C₄- bis C₆-Olefinen mit überwiegend innenständigen Doppelbindungen aus einem C₄ - C₆-Olefin oder C₄ - C₆-Olefinschnitt ohne oder mit bis zu 5 %, vorzugsweise bis zu 2 %, mehrfach ungesättigten Kohlenwasserstoffen, das folgende Schritte umfasst:
a) Hydrierung der gegebenenfalls vorhandenen mehrfach ungesättigten Kohlenwasserstoffe mit Wasserstoff auf eine Restkonzentration von unter 5 ppm
b) Isomerisierung der von vornherein vorhandenen oder der durch die Hydrierung entstandenen Olefine mit endständigen Doppelbindungen zu solchen mit innenständigen Doppelbindungen an einem Kontakt, der mindestens ein Metall der achten Nebengruppe des Periodensystem enthält, in Gegenwart mindestens einer zugesetzten Schwefelverbindung, die mindestens 4 Kohlenstoffatome enthält, und von Wasserstoff
c) Abtrennung der Schwefelverbindungen aus dem Produktgemisch
d) Gegebenenfalls Rückführung der aus dem Produkt abgetrennten Schwefelverbindungen in den Eduktstrom.
e) Gegebenenfalls fraktionierende Aufarbeitung der praktisch schwefelfreien Olefine.

Die Schritte a) und b) können zusammen oder auch getrennt (in getrennten Apparaten) durchgeführt werden.

Eine spezielle Ausführungsform der Erfindung ist ein Verfahren zur Herstellung sowohl eines C₄-Kohlenwasserstoffgemisches, das als Olefine nur 2-Butene enthält, als auch eines Gemisches, das als Olefine Isobuten und gegebenenfalls 1-Buten enthält, aus einem C₄-Schnitt mit maximal 2 % Dienen, das aus folgenden Schritten besteht:
a) Selektivhydrierung des Diengehalts von maximal 2 % auf unter 5 ppm und gleichzeitige Isomerisierung von 1-Buten zu 2-Butenen an einem Kontakt, der mindestens ein Metall der achten Nebengruppe des Periodensystems der Elemente enthält, in Gegenwart einer zugesetzten Schwefelverbindung mit mindestens 4 Kohlenstoffatomen, die nicht mit den Olefinen reagiert und einen höheren Siedepunkt als die Produktolefine hat.
b) Destillative Auftrennung des Reaktionsgemisches, wobei eine Kopffraktion, bestehend aus Isobuten, Isobutan und gegebenenfalls 1-Buten, eine Sumpffraktion mit den Schwefelverbindungen sowie eine weitere Fraktion, die durch Seitenabzug aus dem unteren Teil der Kolonne entnommen wird, mit 2-Butenen und Butan erhalten werden.
c) Rückführung eines Teils der die Schwefelverbindungen enthaltenden Sumpffraktion in den Reaktor.

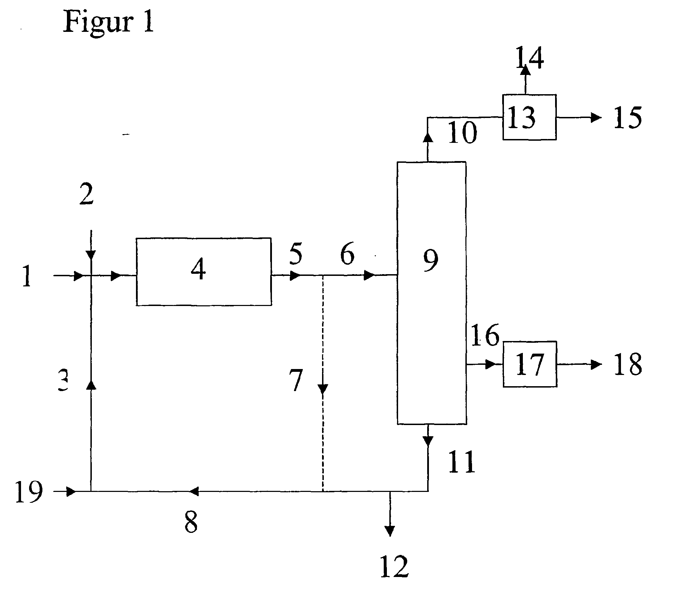

Das erfindungsgemäße Verfahren kann diskontinuierlich oder vorteilhaft kontinuierlich durchgeführt werden. Für die kontinuierliche Durchführung sind verschiedene Verfahrensvarianten möglich. In Figur 1 ist als Beispiel das Blockschema einer Anlage dargestellt, in der das Verfahren für die Aufarbeitung von C₄-Gemischen kontinuierlich durchgeführt werden kann. In den Hydrier- und Isomerisierungsreaktor 4 werden Edukt 1, Wasserstoff 2 und Strom 3, der Schwefelverbindungen enthält, eingespeist. Der Reaktoraustrag 5 wird teilweise oder vollständig in die Destillationskolonne 9 eingeleitet. Eine gegebenenfalls abgetrennte Teilmenge des Reaktoraustrags 5 wird als Strom 7 in den Reaktor 4 zurückgefahren. Das Kopfprodukt 10 der Kolonne 9, das die gesamte Isobutenmenge enthält, wird im Wärmeaustauscher 13 kondensiert. Nach Abtrennen des Abgases 14 wird ein Teil des Kondensates als Rücklauf durch eine nicht gezeichnete Leitung auf den Kopf der Kolonne 9 zurückgeführt. Der andere Teil kann bekannten Verwendungszwecken zugeführt werden. Die 2-Buten-haltige Fraktion 16 wird gasförmig als Seitenabzug im unteren Teil der Kolonne 9 gewonnen. Sie kann direkt oder nach Kondensation im Wärmeaustauscher 17 genutzt werden. Das Sumpfprodukt 11 enthält die Schwefelverbindungen. Dieses kann, gegebenenfalls nach Ausschleusung eines Teilstroms 12 und Ergänzung durch frische Schwefelverbindungen 19, in den Reaktor 4 zurückgeführt werden.

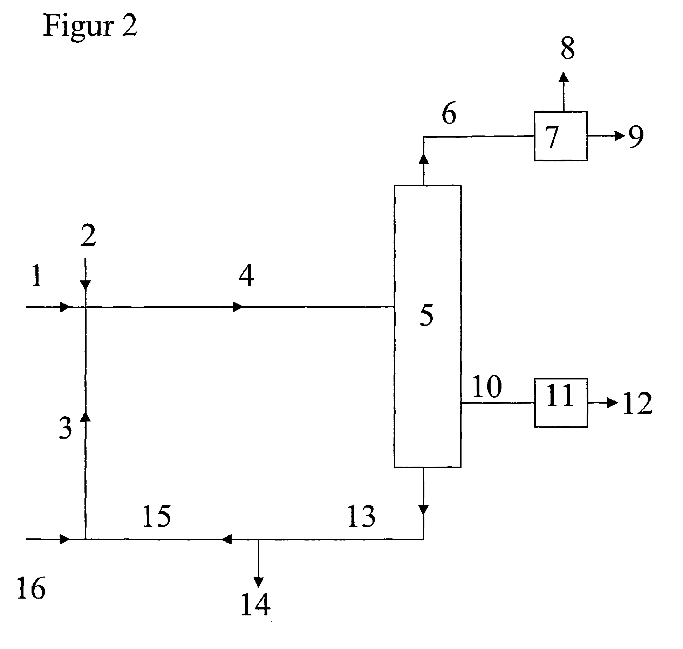

Das Blockschema einer alternativen zweiten kontinuierlich arbeitenden Verfahrensvariante zur Durchführung des Verfahrens nach der Erfindung ist in Figur 2 dargestellt. Edukt 1, Wasserstoff 2 und Schwefelverbindungen 3 werden einer Reaktivkolonne 5 zugeführt. In dieser Kolonne befindet sich eine Gewebepackung, die den Katalysator enthält. Hydrierung, Isomerisierung und Fraktionierung erfolgen darin gleichzeitig. Als Kopfprodukt 6 fällt eine Isobutenfraktion an, die praktisch frei an 1-Buten sein kann. Nach ihrer Kondensation im Wärmeaustauscher 7, Abtrennung von Abgas 8, Rückführung einer Teilmenge auf den Kopf der Kolonne 5 (Rückflussleitung nicht gezeichnet) wird Strom 9 erhalten, der in nachgeschalteten Anlagen genutzt wird. Die 2-Buten-Fraktion 10 (2-Buten, n-Butan) wird gasförmig als Seitenabzug im unteren Teil der Reaktivkolonne 5 gewonnen. Dieser Strom kann, gegebenenfalls nach Kondensation im Wärmeaustauscher 11, der weiteren Verarbeitung zugeführt werden. Das Sumpfprodukt 13 enthält die Schwefelverbindungen. Dieses kann, gegebenenfalls nach Ausschleusung eines Teilstroms 14 und Ergänzung durch frische Schwefelverbindungen 16, in den Reaktor 5 zurückgeführt werden.
Das Blockschema einer dritten kontinuierlich arbeitenden Verfahrensvariante zur Durchführung des Verfahrens nach der Erfindung gleicht Figur 1, mit dem Unterschied, das Apparat 9 eine Reaktivdestillationskolonne ist.

Erfindungsgemäß wird das Verfahren zur Entfernung von mehrfach ungesättigten Kohlenwasserstoffen, insbesondere von Dienen, und Isomerisierung von Olefinen, insbesondere in Kohlenwasserstoffgemischen angewendet. Geeignete Einsatzstoffe dafür sind Olefinschnitte, die weniger als 5 %, bevorzugt weniger als 2 %, an mehrfach ungesättigten Verbindungen enthalten.
Für die Herstellung von C₅- und C₆-Olefinen kommen vor allem Leichtbenzinfraktionen aus Raffinerien und Crackern in Betracht. Technische Einsatzstoffe für die Gewinnung von C₄₋Olefinen nach dem erfindungsgemäßen Verfahren sind schwefelarme oder schwefelfreie C₄₋Schnitte, wie zum Beispiel sogenanntes Raffinat I, gewonnen aus dem C₄-Schnitt eines Steamcrackers durch Butadien-Extraktion, und hydrierter C₄-Schnitt eines Steamcrackers, wobei der größte Teil des Butadiens selektiv zu linearen Butenen hydriert worden ist. Weitere geeignete Edukte sind Olefingemische, die durch Fischer-Tropsch-Synthese erzeugt worden sind. Darüber hinaus können C₄-Olefingemische, die durch Dehydrierung von Butanen oder durch andere technische Prozesse hergestellt worden sind, eingesetzt werden. C₄-Schnitte aus FCC-Einheiten sind im Allgemeinen schwefelreich. Diese können nur dann im erfindungsgemäßen Verfahren eingesetzt werden, wenn sie untypischerweise schwefelarm sind oder wenn sie ganz oder teilweise entschwefelt worden sind. Es ist aber auch möglich, mehr oder weniger reine Monoolefine einzusetzen.
Erfindungsgemäß werden Rohstoffe verwendet, die weniger als 100 ppm, vorzugsweise weniger als 20 ppm Schwefel enthalten. Bei schwefelreicheren Einsatzstoffen muss daher vor der Hydroisomerisierung zumindestens eine Teilentschwefelung durchgeführt werden. Weiterhin gilt für die mit den Einsatzstoff eingeschleppten Schwefelverbindungen, dass sie beziehungsweise die aus ihnen während der Hydrierung und Hydroisomerisierung entstandenen Stoffe aus dem Produkt entfernt werden können. Geschieht ihre Abtrennung durch Destillation, so müssen sie eine andere Siedelage als die Einsatzolefine und Zielprodukte haben.

(Die Begriffe Kontakt und Katalysator werden als weitgehend synonym verwendet, insbesondere, da es sich um Katalysatoren auf Trägern handelt).

Für das erfindungsgemäße Verfahren werden Trägerkatalysatoren eingesetzt, die mindestens ein Metall der achten Nebengruppe des Periodensystems als aktive Komponente enthalten. Ein bevorzugtes Metall ist Palladium. Die Metallkonzentrationen betragen 0,2 - 2,0 % (bezogen auf den Gesamtkatalysator), vorzugsweise 0,5 - 1,0 %. Als Trägermaterialien werden MgO, Al₂O₃, SiO₂, TiO₂, SiO₂/Al₂O₃, CaCO₃ oder Aktiv-Kohle verwendet. Bevorzugte Trägermaterialien sind Al₂O₃, SiO₂ und SiO₂/Al₂O₃.

Im angelieferten frischen Kontakt liegt die aktive Komponente meistens in der oxidischen oder salzartigen Form vor. Daher muss der Kontakt vor Gebrauch mit Wasserstoff oder wasserstoffhaltigen Gasgemischen reduziert werden. Die Reduktion des unreduzierten Katalysators kann in einem separaten Reaktor oder im Hydroisomerisierungsreaktor erfolgen. Dabei geht die aktive Komponente in den metallischen Zustand über.
Der Katalysator kann aber auch bereits reduziert angeliefert und eingesetzt werden.
Der reduzierte Kontakt katalysiert sowohl die Isomerisierung der Monoene als auch die Hydrierung der mehrfach ungesättigten Kohlenwasserstoffe. Seine Aktivität muss so weit herabgesetzt werden, dass zwar weiterhin die Isomerisierung der Monoolefine und Hydrierung der mehrfach ungesättigten Kohlenwasserstoffe erfolgt, die Hydrierung der Monoolefine jedoch weitgehend unterdrückt wird. Erfindungsgemäß wird die Aktivität des Katalysators, der optional schon bei seiner Herstellung vorgeschwefelt worden ist, mit Hilfe von Schwefelverbindungen, insbesondere von bestimmten Thioethern, herabgesetzt und eingestellt. Die Schwefelverbindungen wie insbesondere die Thioether werden reversibel vom Kontakt adsorbiert. Ihre Konzentration an der Kontaktoberfläche ist eine Funktion der Konzentration der Schwefelverbindung im Reaktorzulauf. Die Belegung des Frischkontaktes mit den Schwefelverbindungen geschieht dadurch, dass der Kontakt so lange mit einer Lösung, bestehend aus der Schwefelverbindung und einem für den Kontakt inerten Solvens, beschickt wird, bis die Konzentrationen der Schwefelverbindung im Reaktor-Zufluss und -Abfluss gleich sind. Dadurch wird eine gleichmäßige Belegung des Katalysators über die gesamte Katalysatormenge erreicht. Als Solvens kann beispielsweise ein Kohlenwasserstoff verwendet werden. Zweckmäßig ist es, das Hydriergut selbst hierfür zu verwenden.

Erfindungsgemäß beträgt bei der Umsetzung zur Steuerung der Katalysator-Aktivität und - selektivität der Schwefelgehalt im Reaktorzulauf 1 bis 100 ppm, vorzugsweise 2 bis 20 ppm.

Die dafür eingesetzten Schwefelverbindungen müssen im Produkt und im Edukt löslich sein, sich reversibel an den Kontakt binden und dürfen keine Stoffe bilden, die den Kontakt vergiften.

Da im erfindungsgemäßen Verfahren Produkte mit sehr geringen Schwefelgehalten erzeugt werden, müssen die zugesetzten Schwefelverbindungen und daraus entstandene Schwefelverbindungen destillativ abtrennbar sein.

Es ist bekannt, dass die meisten organischen Schwefelverbindungen unter hydrierenden Bedingungen nicht stabil sind, weil die C-S- oder S-S-Bindung angegriffen wird. So können beispielsweise aus Mercaptanen Kohlenwasserstoffe und Schwefelwasserstoff und aus Dialkyldisulfiden Mercaptane entstehen. Die zugesetzten Schwefelverbindungen und die daraus entstandenen Schwefelverbindungen können miteinander oder mit Olefinen reagieren, sodass sich schwefelhaltige Stoffe mit der gleichen Siedelage wie die Zielprodukte bilden können. Nachteilig ist auch, dass formal Umalkylierungen am Schwefelatom stattfinden können.
Weiterhin ist bekannt, dass einige Schwefelverbindungen mit Kohlenwasserstoffen Azeotrope bilden. So ist beispielsweise ein Azeotrop, bestehend aus Isobutan und Methylmercaptan, mit dem Siedepunkt von -13 °C bekannt (F. R. Brooks, A. C. Nixon, Journal of the American Chemical Society 1953, 75, 480).

Wegen der genannten Schwierigkeiten sind im erfindungsgemäßen Verfahren zur Steuerung der Katalysatoraktivität deshalb nur ausgewählte Schwefelverbindungen geeignet.

Im erfindungsgemäßen Verfahren werden Schwefelverbindungen eingesetzt, die unter den Reaktionsbedingungen als inert anzusehen sind und bei der destillativen Aufarbeitung im Sumpfprodukt verbleiben, also destillativ abgetrennt werden können. Es wurde erfindungsgemäß gefunden, dass bestimmte Thioether mit mindestens 4 Kohlenstoffatomen diese Bedingungen erfüllen. Die beiden C-Gruppen der als geeignet gefundenen Thioether können gleich oder unterschiedlich sowie acyclisch, cyclisch, heterocyclisch oder aromatisch sein. Ebenfalls ist es möglich, dass die eingesetzte Verbindung mehr als eine Thioethergruppe aufweist. Alle Thioether mit mindestens 4 C-Atomen sieden höher als das höchst siedende Monoolefin mit 4 - 6 C-Atomen. Diese Verbindungen können von den Produkten destillativ abgetrennt werden und fallen als Sumpfprodukt an.
Um den Aufwand für die destillative Abtrennung der Schwefelverbindungen gering zu halten, werden bevorzugt Thioether mit insgesamt mindestens 8 C-Atomen eingesetzt. Bei der Hydroisomerisierung eines C₄-Schnittes werden daher bevorzugt Dibutylsulfide, insbesondere Di-n-butylsulfid, eingesetzt. Mit ihnen lässt sich die Kontaktaktivität besonders gut steuern. Weiterhin ergeben sie bei gegebenenfalls auftretenden Umalkylierungen am Schwefelatom wiederum Butylthioether, sodass ihre Wirkung nahezu konstant bleibt und damit auch keine Auswirkung auf die Destillation gegeben ist, was die Steuerung der Umsetzung erleichert.

Optional kann auch ein Gemisch von zwei oder mehreren Thioethern zur Steuerung der Hydroisomerisierung eingesetzt werden.

Die Umsetzung am Katalysator erfolgt in Gegenwart von Wasserstoff. Die Wasserstoffmenge hängt von der Menge an mehrfach ungesättigten Kohlenwasserstoffen, insbesondere Dienen, im Reaktorzufluss ab. Die Menge des Wasserstoffs beträgt das 1- bis 4-fache, insbesondere das 1- bis 3-fache, ganz besonders bevorzugt das 2,0- bis 2,6-fache, der stöchiometrischen Menge, die für die Hydrierung der mehrfach ungesättigten Kohlenwasserstoffe zu Monoolefinen notwendig ist. Dies gilt für den Konzentrationsbereich von 0,1 bis 5 Massen-% an mehrfach ungesättigten Kohlenwasserstoffen im Reaktorzufluss.
Im Konzentrationsbereich von 0 bis 0,1 Massen-% an mehrfach ungesättigten Kohlenwasserstoffen wird die Menge an zudosiertem Wasserstoff auf den Gehalt an Monoolefinen im Reaktorzufluss bezogen. In diesem Konzentrationsbereich wird je Mol Monoolefin 0,002 bis 0,006 Mol, insbesondere 0,003 bis 0,004 Mol Wasserstoff zudosiert.

Die Umsetzung wird im Temperaturbereich von 30 - 150 °C, insbesondere 40 - 120 °C, ganz besonders bevorzugt 80 - 100 °C durchgeführt. Die relativ niedrigen Temperaturen sind besonders vorteilhaft, da sie ein günstiges thermodynamisches Gleichgewicht zwischen Olefinen mit innenständigen und denen mit endständigen Doppelbindungen bedingen. So beträgt beispielsweise in diesem Temperaturbereich das Verhältnis von 2-Butenen zu 1-Buten bei Einstellung des thermodynamischen Gleichgewichts circa 18 : 1 - 16 : 1.

Wegen der niedrigen Siedepunkte der C₄ - C₆-Kohlenwasserstoffe wird die Umsetzung vorzugsweise unter Druck ausgeführt, damit die Reaktionen im Wesentlichen in der flüssigen Phase erfolgen können. Der Druckbereich erstreckt sich von 5 bis 50 bar, vorzugsweise von 10 bis 30 bar (absolut).

Für die Umsetzungen (Isomerisierung oder Hydrierung mit Isomerisierung) können unterschiedliche Verfahrensvarianten gewählt werden. Sie können adiabatisch oder praktisch isotherm, dass heißt mit einem Temperaturanstieg von typischerweise kleiner 10 K, ein- oder mehrstufig durchgeführt werden. Im letzteren Fall kann man alle Reaktoren, zweckmäßig Rohrreaktoren, adiabatisch oder praktisch isotherm oder die einen adiabatisch und die anderen praktisch isotherm betreiben. Weiterhin ist es möglich, das Edukt im geraden Durchgang oder mit Produktrückführung umzusetzen. Die Reaktoren können als Gleichstromreaktoren mit Rieselbett, bevorzugt mit hohen Flüssigkeitsbelastungen betrieben werden. Im Interesse einer hohen Raum-Zeit-Ausbeute werden die Reaktoren vorzugsweise mit hohen Flüssigkeitsbelastungen von 5 - 100 m³, insbesondere von 15 - 30 m³ pro m² Querschnitt des leeren Reaktors und Stunde betrieben. Wird ein Reaktor isotherm und im geraden Durchgang betrieben, so kann die spezifische Katalysatorbelastung (LHSV) Werte zwischen 1 und 20 h⁻¹, vorzugsweise 4 bis 12 h⁻¹, annehmen.

Um Temperaturspitzen im Reaktor, die zur Schädigung des Katalysators und zur Bildung von Nebenprodukten führen könnten, zu vermeiden, ist es zweckmäßig, die Konzentration an endständigen Olefinen im Reaktorzulauf auf höchstens 20 Massen-%, insbesondere höchstens 15 Massen-% zu begrenzen. Liegt der Gehalt an endständigen Olefinen im Edukt über diesen Grenzen, wird dieses verdünnt. Dabei ist es zweckmäßig, das Hydroisomerisat als Verdünnungsmittel einzusetzen.

Aus dem Austrag des Hydroisomerisierungsreaktors werden die Schwefelverbindungen abgetrennt. Dies geschieht beispielsweise durch Adsorption oder vorzugsweise durch Destillation. Die so erhaltenen schwefelfreien Rohprodukte können nach bekannten Verfahren weiterverarbeitet werden, wie zum Beispiel zur Alkylierung zu schwefelarmen Benzinkomponenten.

Bei der destillativen Aufarbeitung in einer oder mehreren Kolonnen werden die Schwefelverbindungen abgetrennt und vorzugsweise die Olefine in mindestens zwei Fraktionen getrennt.
In einer bevorzugten Ausführungsform wird beispielsweise ein C₄₋Hydroisomerisierungsgemisch bei Verwendung nur einer Kolonne in eine Kopf-, Seiten- und Sumpffraktion getrennt. Der Destillationsdruck beträgt dabei 3 bis 20 bar, insbesondere 3 bis 10 bar, ganz besonders 6 bis 8 bar (absolut). Die mittlere Destillationstemperatur liegt zwischen 30 und 130 °C, insbesondere zwischen 30 und 100 °C, ganz besonders bevorzugt zwischen 60 und 70 °C. Das Rücklaufverhältnis liegt zwischen 5 und 30, insbesondere zwischen 10 und 20 und ganz besonders bevorzugt zwischen 15 und 20.
Der Seitenabzug erfolgt von einem Boden, der 1 bis 15 theoretische Böden oberhalb des Sumpfes gelegen ist.
Eine weitere Ausführungsvariante besteht darin, dass die Umsetzung (Isomerisierung oder Hydrierung und Isomerisierung) und Auftrennung gleichzeitig in einer Reaktivkolonne, die Einbauten und Katalysator enthält, durchgeführt wird. Ist beispielsweise ein C₄-Schnitt das Edukt, wird ein Druck von 6 bis 10 bar und eine Temperatur von 60 bis 100 °C angewendet. Dabei kann ein nahezu Isobuten-freies Kopfprodukt gewonnen werden.

Weiterhin ist es möglich, den größten Teil der Umsetzungen in einem oder mehreren Reaktor(en) ablaufen zu lassen und den restlichen Teil der Umsetzungen zusammen mit der Stofftrennung in einer Reaktivdestillationskolonne durchzuführen.

Die im Sumpf verbleibenden Schwefelverbindungen können in den Reaktor zurückgeführt werden. Ein Teil des Sumpfproduktes kann ausgeschleust werden, um ein Ansteigen des Schwefelgehaltes durch die gegebenenfalls mit dem Edukt eingebrachten Schwefelverbindungen zu verhindern oder Schwefelverbindungen mit unerwünschten Eigenschaften aus dem Prozess zu entfernen. Andererseits können dem Rückführungsstrom Schwefelverbindungen mit definierten Eigenschaften zugesetzt werden. Es besteht daher die Möglichkeit der Steuerung der Katalysatoraktivität durch den Schwefelgehalt und die Art der Schwefelverbindungen im Reaktorzulauf.

Nur wenn das Edukt hochsiederfrei ist und während der Hydroisomerisierung oder Hydrierung und Hydroisomerisierung keine Hochsieder entstehen, können die Schwefelverbindungen vollständig zurückgeführt werden. Fallen dagegen Hochsieder, ausgenommen Schwefelverbindungen, im Destillationssumpf an, so werden die Schwefelverbindungen zusammen mit dem Hochsieder ausgeschleust, sodass eine wirtschaftliche Schwefelrückführung nicht möglich ist. Wenn beispielsweise ein schwefelfreier C₄₋Kohlenwasserstoffstrom mit 1 Massen-% Hochsieder (Kohlenwasserstoffe mit mehr als 4 C-Atomen) erfindungsgemäß hydroisomerisiert beziehungsweise hydriert und hydroisomerisiert wird, wobei der Gehalt an Hochsiedern im Hydroisomerisierungsreaktor auf 5 Massen-% begrenzt sein soll, werden zusammen mit den Hochsiedern etwa 20 % der zugesetzten Schwefelverbindungen ausgeschleust.

Der Rückführungsgrad der Schwefelverbindungen kann daher unter anderem wegen der Zusammensetzung des Eduktes über einen Bereich von 100 bis 10 % variieren. Typischerweise liegt er bei der Hydroisomerisierung beziehungsweise Hydrierung und Hydroisomerisierung von C₄-Kohlenwasserstoffgemischen bei 100 bis 50 %, insbesondere bei 95 bis 60 %.

Besteht die Sumpffraktion aus einem Gemisch aus Schwefelverbindungen, Hochsiedern, Produkt und/oder Edukt, so ist es zweckmäßig, Edukte und Produkte in einer weiteren Destillation von den auszuschleusenden Hochsiedern abzutrennen.

Wird ein C₄-Schnitt hydroisomerisiert beziehungsweise hydriert und hydroisomerisiert und destillativ aufgearbeitet, fällt ein Kopfprodukt an, das Isobuten, 1-Buten und Isobutan und maximal 1 Massen-ppm, insbesondere 500 Massen-ppb, ganz besonders 100 Massen-ppb Schwefel enthält. Dieses Gemisch kann in einer weiteren Kolonne in Isobutan und Olefine aufgetrennt werden. Das Olefingemisch kann nach bekannten Verfahren weiterverwendet werden, beispielsweise zur Herstellung von MTBE. Wird die Hydroisomerisierung in einer Reaktivkolonne durchgeführt, kann ein praktisch 1-Buten-freies Isobuten erhalten werden. Dieses kann für Reaktionen genutzt werden, bei denen 1-Buten stört.

Die 2-Butene, die Butan enthalten, werden gasförmig als Seitenstrom abgezogen. Der Schwefelgehalt liegt unter 500 Massen-ppb, insbesondere unter 100 Massen-ppb, ganz besonders unter 50 Massen-ppb und eignet sich dann insbesondere für die Herstellung von Alkylatbenzin. Nach Entfernung dieser Schwefelspuren, beispielsweise durch Adsorption an einem im Festbett angeordneten Adsorptionsmaterial wie zum Beispiel einem Molsieb, kann das 2-Butengemisch für extrem schwefelempfindliche chemische Reaktionen wie zum Beispiel die Oligomerisierung an nickelhaltigen Katalysatoren eingesetzt werden.

Das erfindungsgemäße Verfahren hat insbesondere also folgende Vorteile:
- Mehrfach ungesättigte Kohlenwaserstoffe werden bis auf einen Gehalt unter 5 ppm hydriert.
- Selbst bei hohen Monoenkonzentrationen im Edukt treten, wenn überhaupt, nur geringe Monoolefinverluste durch Überhydrierung auf.
- Das Isomerenverhältnis der Monoolefine liegt in der Nähe des thermodynamischen Gleichgewichts.
- Die gewonnenen Olefine beziehungsweise deren Gemische können vorteilhaft für die Herstellung schwefelarmer Produkte eingesetzt werden.
- Die Schwefelkomponente wird nahezu vollständig zurückgewonnen und verursacht daher nur geringe Kosten.
- Die Aktivität des Katalysators kann den Betrieberfordernissen angepasst werden.

Die folgenden Beispiele sollen die Erfindung erläutern, ohne ihre Anwendungsbreite einzuschränken, die sich aus der Beschreibung und den Patentansprüchen ergibt.

Die in den Beispielen angegeben Konzentrationen werden unter Anwendung folgender Analysenmethoden ermittelt:
Bestimmung der C₄-Isomeren:
   - gaschromatographische Untersuchung (100 % Normierung)
   - Detektor: FID
   - Trennsäule: 50 m PLOT Al₂O₃/Na₂SO₄; 0,32 mm ID; 5 µm Filmdicke (Fa. Chrompack)
   - Ofentemperatur: 125 °C isotherm
   - Detektortemperatur: 140 °C
   - Injektortemperatur: 200 °C
   - Trägergas: Helium 3,0 ±0,5 ml/Min.
   - Säulenvordruck: 180 ± 10 kPa
   - Split: 200 ±20 ml/Min.
   - Einspritzmenge: 50 µl

Die Bestimmung von 1,3-Butadien im Spurenbereich (Nachweisgrenze: 5 mg/kg) erfolgt wie die Bestimmung der C₄-Isomeren, allerdings mit einer Einspritzmenge von 1,0 ml.

Bestimmung des Schwefels im Spurenbereich (Nachweisgrenze: 5 - 10 µg/kg):
- gaschromotographische Untersuchung (externe Standardmethode)
- Detektor: FPD
- Trennsäule: 25 m CP - SIL 5 CB; 0,53 mm ID; 5 µm Filmdicke (Chrompack)
- Ofentemperatur: 35 °C; 1 Min; 20 K/Min; 190 °C; 9 Min
- Detektortemperatur: 160 °C
- Injektortemperatur: 180 °C
- Trägergas: Helium 15,0 ± 2,0 ml/Min
- Säulenvordruck: 60 ± 10 kPa
- Splitlos
- Einspritzmenge: 5 µl (flüssig, bei - 80 °C kondensiert)

### Beispiel 1 (Vergleichsbeispiel):

Die Hydroisomerisierung eines C₄-Rohstoffes wird in der Flüssigphase an einem Festbettkatalysator, der 1 % Palladium auf Al₂O₃ enthält und analog EP-A-0 636 667 hergestellt wurde, durchgeführt. Der Rohstoff wird auf 55 °C vorgeheizt und der Reaktor im geraden Durchgang adiabatisch gefahren. Die Reaktion findet bei einer mittleren Temperatur von 80 °C statt. Die spezifische Katalysatorbelastung (LHSV) (Volumen Edukt je Volumen Katalysator je Zeit) beträgt 12 1/(1* h), das molare Wasserstoff/Dien-Verhältnis 2,0. Der Rohstoff enthält keinen Schwefel. Die Tabelle 1 zeigt die Zusammensetzung des Reaktorzulaufs und -austrages.

| Tabelle1 Komponente (Ma-%) | Zulauf (Ma-%) | Austrag (Ma-%) |
|---|---|---|
| 1,3-Butadien | 0,4 | 0 |
| Isobuten | 25,2 | 25,2 |
| 1-Buten | 38,2 | 5,6 |
| 2-Butene | 26,7 | 58,4 |
| Isobutan | 3,2 | 3,2 |
| n-Butan | 7,0 | 7,6 |

### Beispiel 2 (Vergleichsbeispiel):

Die Hydroisomerisierung wird wie in Beispiel 1 durchgeführt. Dem Rohstoff wird jedoch Schwefel in Form von Dimethyldisulfid zugegeben, sodass die Schwefelkonzentration am Reaktorzulauf 3 ppm beträgt. Tabelle 2 zeigt die Zusammensetzung des Reaktoraustrages. Die Analyse des Produktes auf Schwefelkomponenten ergibt ein Gemisch verschiedener schwefelhaltiger Verbindungen. Neben einigen nicht identifizierten Komponenten werden Dimethyldisulfid, Methylbutylsulfid und Dibutylsulfid nachgewiesen. Die Konzentrationen liegen als Schwefel berechnet zwischen 20 ppb und 700 ppb. Die Schwefelverbindungen können destillativ (wie in Beispiel 4 und 5 beschrieben) nicht abgetrennt werden.

**Tabelle 2**

| Komponente (Ma-%) | Austrag (Ma-%) |
|---|---|
| 1,3-Butadien | 0 |
| Isobuten | 25,2 |
| 1-Buten | 3,4 |
| 2-Butene | 60,7 |
| Isobutan | 3,2 |
| n-Butan | 7,5 |

Das Beispiel verdeutlicht damit den positiven Einfluß der Schwefelzugabe bei der Hydroisomerisierung, zeigt jedoch auch die Schwierigkeiten bei der Abtrennung der Schwefelverbindungen auf.

### Beispiel 3 (erfindungsgemäß):

Die Hydroisomerisierung wird wie in Beispiel 1 durchgeführt. Dem Rohstoff wird jedoch Schwefel in Form von Di-n-butylsulfid zugegeben, sodass die Schwefelkonzentration am Reaktorzulauf 3 ppm beträgt. Tabelle 3 zeigt die Zusammensetzung des Reaktoraustrages. Die Analyse des Produktes auf Schwefelkomponenten ergibt nur eine Schwefelkomponente, nämlich Di-n-butylsulfid. Zulauf- und Austragskonzentration dieser Schwefelkomponente sind identisch.

**Tabelle 3**

| Komponente (Ma-%) | Austrag (Ma-%) |
|---|---|
| 1,3-Butadien | 0 |
| Isobuten | 25,2 |
| 1-Buten | 3,3 |
| 2-Butene | 60,9 |
| Isobutan | 3,2 |
| n-Butan | 7,4 |

Das Beispiel belegt den vorteilhaften Einsatz der Schwefelkomponente in Form von Di-n-butylsulfid, das im Reaktor nicht zu anderen Schwefelkomponenten umgesetzt wird und destillativ vom C₄-Schnitt getrennt werden kann (siehe hierzu Beispiel 4 und 5).

### Beispiel 4 (Erfindungsgemäße Abtrennung und Rückführung der Schwefelverbindung)

Der Reaktoraustrag aus Beispiel 3 wird destillativ aufgetrennt. Die Aufgabe, einerseits das Isobuten von den 2-Butenen zu trennen und andererseits die als Schwersieder anfallenden 2-Butene schwefelfrei zu erhalten, wird folgendermaßen gelöst:
Einer Destillationskolonne mit ca. 150 theoretischen Böden wird der Reaktoraustrag im oberen Drittel (Boden 45 von oben) zugeführt. Die isobutenhaltige Fraktion wird am Kopf der Kolonne abgezogen, kondensiert und teilweise zum Kopf der Kolonne zurückgeführt (Rücklauf). Ein 2-Buten-reicher Produktstrom wird im Abtriebsteil der Kolonne dampfförmig entnommen (Entnahme-Boden: ca. Boden 1 bis 15 von unten) und außerhalb der Kolonne kondensiert. Die schwersiedende Schwefelkomponente reichert sich im Sumpfprodukt der Kolonne an. Das Sumpfprodukt kann (eventuell nach der Abtrennung schwersiedender Kohlenwasserstoffe) erfindungsgemäß zum Zulauf des Hydroisomerisierungsreaktor zurückgefahren werden, sodass sich ein geschlossener Schwefelkreislauf ergibt.

**Destillationsbedingungen:**

| | |
|---|---|
| Anzahl theoretischer Böden: | 150 |
| Kolonnendurchmesser: | 200 mm |
| Kopfdruck: | 6 bar |
| Sumpftemperatur: | 68 °C |
| Kopftemperatur: | 49 °C |
| Rücklaufverhältnis: | 16 |
| Zulaufstrom: | 5000 g/h |
| Sumpfentnahme: | 46 g/h |
| Destillatstrom: | 1636 g/h |
| Seitenstromabnahme: | 3318 g/h |

**Tabelle 4 zeigt die Zusammensetzung der verschiedenen Ströme bei der Destillation.**

| Komponente | Zulauf | Destillat | Sumpf | Seitenstrom |
|---|---|---|---|---|
| Isobuten (Ma-%) | 25,2 | 76,8 | 0,04 | 0,2 |
| 1-Buten (Ma-%) | 3,3 | 10,0 | -/- | 0,05 |
| 2-Butene (Ma-% | 60,9 | 0,6 | 83,7 | 90,1 |
| Isobutan (Ma-%) | 3,2 | 9,8 | -/- | -/- |
| n-Butan (Ma-%) | 7,4 | 2,8 | 4,6 | 9,7 |
| S (ppm) | 3 | -/- | 322 | -/- |

| | | | | |
|---|---|---|---|---|
| -/- bedeutet nicht nachweisbar, Schwefel liegt als Di-n-butylsulfid vor | | | | |

Dieses Beispiel belegt, dass die Schwefelverbindungen gut abgetrennt werden, sodass praktisch schwefelfreie Produkte gewonnen werden.

### Beispiel 5 (erfindungsgemäße Abtrennung und Rückführung der Schwefelverbindungen)

Obwohl eine Schwefelkonzentration von 3 ppm im Zulauf für die Hydroisomerisierung als ausreichend ermittelt wurde, wird dieses Beispiel für eine Schwefelkonzentration von 20 ppm im Zulauf zur Kolonne angegeben, um die Leistungsfähigkeit dieser Art der Schwefelabtrennung zu belegen. Abgesehen vom Schwefelgehalt entspricht die Zusammensetzung des Kolonnenzulaufes der des im Beispiel 4. Destillationsapparatur und Destillationsbedingungen sind die gleichen wie in Beispiel 4.

**Tabelle 5, Zusammensetzung der verschiedenen Ströme bei der Destillation.**

| Komponente | Zulauf | Destillat | Sumpf | Seitenstrom |
|---|---|---|---|---|
| Isobuten (Ma-%) | 25,2 | 76,8 | 0,04 | 0,2 |
| 1-Buten (Ma-%) | 3,3 | 10,0 | -/- | 0,05 |
| 2-Butene (Ma-% | 60,9 | 0,6 | 83,7 | 90,1 |
| Isobutan (Ma-%) | 3,2 | 9,8 | -/- | -/- |
| n-Butan (Ma-%) | 7,4 | 2,8 | 4,6 | 9,7 |
| S (ppm) | 20 | 0,006 | 2152 | 0,03 |

| | | | | |
|---|---|---|---|---|
| Schwefel liegt als Di-n-butylsulfid vor | | | | |

Dieses Beispiel Beispiel zeigt, dass auch bei höheren Schwefelkonzentrationen im Kolonnenzulauf Produkte mit geringen Schwefelkonzentrationen erhalten werden.

## Patentansprüche

1. Verfahren zur Hydroisomerisierung von C₄- bis C₆-Olefinen oder olefinhaltigen Schnitten mit C₄- bis C₆-Olefinen oder zur Hydroisomerisierung eines Gemisches aus C₄- bis C₆₋Olefinen unter gleichzeitiger Hydrierung von darin bis zu 5 % enthaltenen mehrfach ungesättigten Kohlenwasserstoffen an einem Kontakt, der ein Element der achten Nebengruppe des Periodensystems der Elemente enthält,
**dadurch gekennzeichnet, dass**
die Umsetzung in Gegenwart mindestens einer zugesetzten Schwefelverbindung, die mindestens 4 Kohlenstoffatome enthält, durchgeführt wird, wobei der Reaktorzufluss nur solche Schwefelverbindungen enthält, die und/oder die aus ihnen während der Hydroisomerisierung entstandenen schwefelhaltigen Verbindungen destillativ von den Olefinen abgetrennt werden können.

2. Verfahren nach Anspruch 1,
**dadurch gekennzeichnet,**
**dass** man einen Träger-Katalysator einsetzt, der 0,2 % bis 2,0 % Palladium, bezogen auf das Gesamtgewicht des Träger-Katalysators, enthält.

3. Verfahren nach Anspruch 1 oder 2,
**dadurch gekennzeichnet,**
**dass** man als Katalysatorträger Aluminiumoxid (Al₂O₃), Siliciumdioxid (SiO₂) oder Silicaaluminiumoxid (SiO₂/Al₂O₃) verwendet.

4. Verfahren nach Anspruch 1,
**dadurch gekennzeichnet,**
**dass** der Schwefelgehalt im Reaktorzufluss 1 ppm bis 100 ppm beträgt.

5. Verfahren nach Anspruch 1 oder 4,
**dadurch gekennzeichnet,**
**dass** Thioether in den Reaktorzufluss eingespeist werden.

6. Verfahren nach Anspruch 5,
**dadurch gekennzeichnet,**
**dass** Thioether mit mindestens 8 Kohlenstoffatomen in den Reaktorzufluss eingespeist werden.

7. Verfahren nach mindestens einem der Ansprüche 1 bis 6,
**dadurch gekennzeichnet,**
**dass** Dibutylsulfide in den Reaktorzufluss eingespeist werden.

8. Verfahren nach mindestens einem der Ansprüche 1 bis 7,
**dadurch gekennzeichnet,**
**dass** bei einer Konzentration von 0,1 bis 5 Massen-% an mehrfach ungesättigten Kohlenwasserstoffen im Reaktorzufluss das 1- bis 4-fache der stöchiometrischen Menge an Wasserstoff eingespeist wird, die zur Selektivhydrierung der mehrfach ungesättigten Kohlenwasserstoffe zu den entsprechenden Monoolefinen notwendig ist, oder dass bei einer Konzentration von 0 bis 0,1 Massen-% an mehrfach ungesättigten Kohlenwasserstoffen im Reaktorzufluss mindestens 0,002 bis 0,006 Mol Wasserstoff je Mol Monoolefin im Reaktorzufluss zudosiert wird.

9. Verfahren nach mindestens einem der Ansprüche 1 bis 7,
**dadurch gekennzeichnet,**
**dass** bei einer Konzentration von 0,1 bis 5 Massen-% an mehrfach ungesättigten Kohlenwasserstoffen im Reaktorzufluss das 2- bis 2,6-fache der stöchiometrischen Menge an Wasserstoff eingespeist wird, die zur Selektivhydrierung der mehrfach ungesättigten Kohlenwasserstoffe zu den entsprechenden Monoolefinen notwendig ist oder dass bei einer Konzentration von 0 bis 0,1 Massen-% an mehrfach ungesättigten Kohlenwasserstoffen im Reaktorzufluss mindestens 0,002 bis 0,006 Mol Wasserstoff je Mol Monoolefin im Reaktorzufluss zudosiert wird.

10. Verfahren nach Anspruch 1,
**dadurch gekennzeichnet,**
**dass** der Hydroisomerisierungsaustrag in mindestens eine Olefinfraktion und eine Fraktion, die die Schwefelverbindungen enthält, aufgetrennt wird.

11. Verfahren nach einem der vorhergehenden Ansprüche,
**dadurch gekennzeichnet,**
**dass** ein Teil der bei der Destillation abgetrennten Schwefelverbindungen in den Reaktorzufluss eingespeist wird.

12. Verfahren nach einem der vorhergehenden Ansprüche,
**dadurch gekennzeichnet,**
**dass** der Hydroisomerisierungsaustrag in eine schwefelhaltige Fraktion und mindestens zwei Olefinfraktionen getrennt wird.

13. Verfahren nach Anspruch 10,
**dadurch gekennzeichnet,**
**dass** die Fraktionierung in einer einzigen Destillationskolonne durchgeführt wird.

14. Verfahren nach Anspruch 10,
**dadurch gekennzeichnet,**
**dass** die Fraktionierung in mindestens zwei Destillationskolonnen durchgeführt wird.

15. Verfahren nach Anspruch 12,
**dadurch gekennzeichnet,**
**dass** ein C₄-Hydroisomerisierungsgemisch in eine Leichtsiederfraktion, die Isobuten und gegebenenfalls 1-Buten und Isobutan enthält, eine Mittelfraktion mit 2-Butenen und gegebenenfalls n-Butan und eine Hochsiederfraktion mit den Schwefelverbindungen getrennt wird.

16. Verfahren nach Anspruch 12,
**dadurch gekennzeichnet,**
**dass** ein C₄-Hydroisomerisierungsgemisch in einer Kolonne in eine Kopffraktion, die Isobuten und gegebenenfalls 1-Buten und Isobutan enthält, eine Seitenfraktion mit 2-Butenen und gegebenenfalls n-Butan und eine Hochsiederfraktion mit den Schwefelverbindungen getrennt wird.

17. Verfahren nach Anspruch 16,
**dadurch gekennzeichnet,**
**dass** die Seitenfraktion dampfförmig entnommen wird.

18. Verfahren nach Anspruch 15,
**dadurch gekennzeichnet,**
**dass** der Seitenstrom von einem Boden, der 1 bis 15 theoretische Böden oberhalb des Sumpfes gelegen ist, abgezogen wird.

19. Verfahren nach mindestens einem der vorhergehenden Ansprüche,
**dadurch gekennzeichnet,**
**dass** in den beiden Olefinfraktionen der Schwefelgehalt kleiner als 500 ppb ist.

## Claims

1. A process for the hydroisomerization of a C₄-C₆₋olefin or olefinic cut containing C₄-C₆-olefins or for the hydroisomerization of a mixture of C₄-C₆₋olefins with simultaneous hydrogenation of up to 5% of polyunsaturated hydrocarbon contained therein over a catalyst containing an element of the eighth transition group of the Periodic Table, **characterized in that** the reaction is conducted in the presence of at least one added sulphur compound which contains at least 4 carbon atoms, the reactor feed only containing sulphur compounds which can be separated from the olefin by distillation and/or which on hydroisomerization give rise to sulphurous compounds which can be separated from the olefin by distillation.

2. A process according to claim 1, **characterized in that** a supported catalyst is used which contains from 0.2 to 2.0% palladium, based on the total weight of the supported catalyst.

3. A process according to either of claims 1 and 2, **characterized in that** aluminium oxide (Al₂O₃), silicon dioxide (SiO₂) or silica-aluminium oxide (SiO₂/Al₂O₃) is used as the catalyst support.

4. A process according to claim 1, **characterized in that** the sulphur content in the reactor feed is from 1 to 100 ppm.

5. A process according to either of claims 1 and 4, **characterized in that** thioether is introduced into the reactor feed.

6. A process according to claim 5, **characterized in that** thioether having at least 8 carbon atoms is introduced into the reactor feed.

7. A process according to at least one of claims 1 to 6, **characterized in that** dibutyl sulphides are introduced into the reactor feed.

8. A process according to at least one of claims 1 to 7, **characterized in that** from 1 to 4 times the stoichiometric quantity of hydrogen required for selective hydrogenation of the polyunsaturated hydrocarbon to give the corresponding monoolefin is introduced when a concentration of from 0.1 to 5 mass% of polyunsaturated hydrocarbon is present in the reactor feed, or **in that** at least 0.002-0.006 mol of hydrogen per mole of monoolefin in the reactor feed is metered in when a concentration of from 0 to 0.1 mass% of polyunsaturated hydrocarbon is present in the reactor feed.

9. A process according to at least one of claims 1 to 7, **characterized in that** from 2 to 2.6 times the stoichiometric quantity of hydrogen required for selective hydrogenation of the polyunsaturated hydrocarbon to give the corresponding monoolefin is introduced when a concentration of from 0.1 to 5 mass% of polyunsaturated hydrocarbon is present in the reactor feed, or **in that** at least 0.002-0.006 mol of hydrogen per mole of monoolefin in the reactor feed is metered in when a concentration of from 0 to 0.1 mass% of polyunsaturated hydrocarbon is present in the reactor feed.

10. A process according to claim 1, **characterized in that** the hydroisomerization effluent is separated into at least one olefin fraction and a fraction which contains the sulphur compounds.

11. A process according to any one of the preceding claims, **characterized in that** part of the sulphur compounds separated off by distillation is fed into the reactor feed.

12. A process according to any one of the preceding claims, **characterized in that** the hydroisomerization effluent is separated into a sulphurous fraction and at least two olefinic fractions.

13. A process according to claim 10, **characterized in that** the fractionation is carried out in a single distillation column.

14. A process according to claim 10, **characterized in that** the fractionation is carried out in at least two distillation columns.

15. A process according to claim 12, **characterized in that** a C₄-hydroisomerization mixture is separated into a low-boiler fraction containing isobutene and possibly 1-butene and isobutane, a middle fraction comprising 2-butenes and possibly n-butane and a high-boiler fraction comprising the sulphur compounds.

16. A process according to claim 12, **characterized in that** a C₄-hydroisomerization mixture is separated in a column into a top fraction, which contains isobutene and possibly 1-butene and isobutane, a side fraction comprising 2-butenes and possibly n-butane, and a high-boiler fraction comprising the sulphur compounds.

17. A process according to claim 16, **characterized in that** the side fraction is withdrawn in vaporous form.

18. A process according to claim 15, **characterized in that** the side stream is withdrawn from a plate located from 1 to 15 theoretical plates above the bottom.

19. A process according to at least one of the preceding claims, **characterized in that** the sulphur content of both the olefin fractions is smaller than 500 ppb.

## Revendications

1. Procédé d'hydroisomérisation d'oléfines en C₄ à C₆ ou de coupes contenant des oléfines comportant des oléfines en C₄ à C₆, ou d'hydroisomérisation d'un mélange d'oléfines en C₄ à C₆, avec hydrogénation simultanée d'hydrocarbures plusieurs fois insaturés qui s'y trouvent contenus dans une proportion allant jusqu'à 5 % sur un contact qui contient un élément du huitième groupe secondaire du Système Périodique des Eléments,
**caractérisé en ce que**
la réaction est effectuée en présence d'au moins un composé soufré ajouté, qui contient au moins 4 atomes de carbone, l'alimentation du réacteur ne contenant que des composés soufrés qui peuvent être séparés des oléfines par distillation et /ou les composés soufrés formés à partir de ceux-ci pendant l'hydroisomérisation.

2. Procédé selon la revendication 1,
**caractérisé en ce qu'**
on utilise un ensemble support - catalyseur qui contient 0,2 % à 2,0 % de palladium, rapporté au poids total du support-catalyseur.

3. Procédé selon la revendication 1 ou 2,
**caractérisé en ce qu'**
on utilise comme support de catalyseur de l'oxyde d'aluminium (Al₂O₃), du dioxyde de silicium (SiO₂) ou un mélange silice-oxyde d'aluminium (SiO₂ / Al₂O₃).

4. Procédé selon la revendication 1,
**caractérisé en ce que**
la teneur en soufre, dans l'arrivée du réacteur, est de 1 ppm à 100 ppm.

5. Procédé selon la revendication 1 ou 4,
**caractérisé en ce qu'**
un thioéther est introduit dans l'alimentation du réacteur.

6. Procédé selon la revendication 5,
**caractérisé en ce qu'**
un thioéther comportant au moins 8 atomes de carbone est introduit dans l'alimentation du réacteur.

7. Procédé selon au moins une des revendications 1 à 6,
**caractérisé en ce que**
des sulfures de dibutyle sont introduits dans l'alimentation du réacteur.

8. Procédé selon au moins une des revendications 1 à 7,
**caractérisé en ce que**
pour une concentration de 0,1 à 5 % en masse d'hydrocarbures plusieurs fois insaturés, on introduit dans l'alimentation du réacteur, 1 à 4 fois la quantité stoechiométrique d'hydrogène nécessaire pour l'hydrogénation sélective des hydrocarbures plusieurs fois insaturés en monooléfines correspondantes, ou pour une concentration de 0 à 0,1 % en masse d'hydrocarbures plusieurs fois insaturés, on ajoute, dans l'alimentation du réacteur, au moins 0,002 à 0,006 mole d'hydrogène par mole de monooléfine.

9. Procédé selon au moins une des revendications 1 à 7,
**caractérisé en ce que**
pour une concentration de 0,1 à 5 % en masse d'hydrocarbures plusieurs fois insaturés, on introduit dans l'alimentation du réacteur, 2 à 2,6 fois la quantité stoechiométrique d'hydrogène nécessaire pour l'hydrogénation sélective des hydrocarbures plusieurs fois insaturés en monooléfines correspondantes, ou pour une concentration de 0 à 0,1 % en masse d'hydrocarbures plusieurs fois insaturés, on ajoute, dans l'alimentation du réacteur, au moins 0,002 à 0,006 mole d'hydrogène par mole de monooléfine.

10. Procédé selon la revendication 1,
**caractérisé en ce que**
le produit de l'hydroisomérisation est séparé en au moins une fraction d'oléfines et une fraction qui contient les composés soufrés.

11. Procédé selon au moins une des revendications précédentes,
**caractérisé en ce qu'**
une partie des composés soufrés séparés au cours de la distillation est introduite dans l'alimentation du réacteur.

12. Procédé selon au moins une des revendications précédentes,
**caractérisé en ce que**
le produit de l'hydroisomérisation est séparé en une fraction contenant du soufre et au moins deux fractions d'oléfines.

13. Procédé selon la revendication 10,
**caractérisé en ce que**
le fractionnement est effectué dans une colonne de distillation unique.

14. Procédé selon la revendication 10,
**caractérisé en ce que**
le fractionnement est effectué dans au moins deux colonnes de distillation.

15. Procédé selon la revendication 12,
**caractérisé en ce que**
le mélange d'hydroisomérisation en C₄ est séparé en une fraction de composés à bas point d'ébullition qui contient de l'isobutène et, le cas échéant, du 1-butène et de l'isobutane, une fraction moyenne contenant des 2-butènes et, le cas échéant, du n-butane, et une fraction de composés à haut point d'ébullition contenant les composés soufrés.

16. Procédé selon la revendication 12,
**caractérisé en ce qu'**
un mélange d'hydroisomérisation en C₄ est séparé dans une colonne en une fraction de tête qui contient de l'isobutène et, le cas échéant, du 1-butène et de l'isobutane, une fraction latérale avec des 2-butènes et, le cas échéant, du n-butane, et une fraction de composés à haut point d'ébullition contenant les composés soufrés.

17. Procédé selon la revendication 16,
**caractérisé en ce que**
la fraction latérale est prélevée sous forme de vapeur.

18. Procédé selon la revendication 15,
**caractérisé en ce que**
le courant latéral est prélevé à partir d'un plateau qui est placé 1 à 15 plateaux théoriques au-dessus du fond de colonne.

19. Procédé selon au moins une des revendications précédentes,
**caractérisé en ce que**
dans les deux fractions d'oléfines, la teneur en soufre est inférieure à 500 ppb.
